# EUROPEAN PATENT SPECIFICATION

(11) **EP 1 602 338 B1**
(45) Date of publication and mention of the grant of the patent: **07.12.2011**
(21) Application number: 05019834.0
(22) Date of filing: 02.10.1996
(51) Int. Cl.: A61B 18/14

(54) **Fluid-assisted electrocautery device**
Elektrokauterisationsvorrichtung mit Hilfe einer Flüssigkeit
Dispositif d'électrocautérisation assisté par fluide

(43) Date of publication of application: 07.12.2005
(62) Divisional of application: 96936132.8
(73) Proprietor: Medtronic, Inc., Minneapolis, MN 55432-5604 (US)
(72) Inventor: Mulier, Peter M.J., Stillwater, MN 55082 (US); Hoey, Michael F., Shorview, MN 55126 (US)
(74) Representative: Hughes, Andrea Michelle

(56) References cited:
- WO-A-96/07360
- US-A- 5 195 959
- US-A- 5 462 521

## Description

This invention relates generally to the field of medical instruments, and more particularly relates to an electrocautery device.

Various types of electrocautery devices for incising and cauterizing body tissue are known and used in the medical field. Typically, such devices include a conductive blade or needle which serves as one electrode in an electrical circuit which is completed via a grounding electrode coupled to the patient. Incision of tissue is accomplished by applying a source of electrical energy (most commonly, a radio-frequency generator) to the blade. Upon application of the blade to the tissue, a voltage gradient is created, thereby inducing current flow and related heat generation at the point of contact. With sufficiently high levels of electrical energy, the heat generated is sufficient to cut the tissue and, advantageously to simultaneously cauterize severed blood vessels.

It is widely recognized in the prior art that the often substantial amount of smoke produced by electrocauterization of tissue is at least unpleasant, and in some cases distracting or even hazardous to the operator and other attending medical personnel. As a result, it has been proposed, and is common, to provide an electrocautery device with smoke-aspirating capabilities, such that the smoke produced from electrocauterization is quickly withdrawn from the area of incision. Smoke aspiration may be accomplished by providing, in the handle of the electrocautery device near the electrocautery blade/electrode, an inlet port to be coupled to a vacuum or suction source. Examples of this are described in U.S. Patent No. 4,307,720 to Weber, Jr., entitled "Electrocautery Apparatus and Method and Means for Cleaning the Same;" in U.S. Patent No. 5,242,442 to Hirschfeld, entitled "Smoke Aspirating Electrosurgical Device;" and in U.S. Patent No. 5.269,781 to Hewell, entitled "Suction Assisted Electrocautery Unit."

It has also been recognized in the prior art that the accumulation of coagulated blood, tissue rubble, and other debris on the electrode/blade of an electrocautery device can present a problem for the operator, necessitating the periodic cleaning of the blade, e.g., by wiping the blade over sterilized gauze or the like. This is generally regarded as undesirable, since the need to clean the electrode/blade tends to interrupt the incision procedure and increases the risks associated with contamination of the blade or the incision, damage to the blade, injury to the operator, and the like. To address this problem, it has been proposed in the prior art to provide an electrocautery instrument in which the electrode/blade is in slidable engagement with the instrument's handle, such that when the blade is retracted into the hand, any adhering debris automatically scraped off onto the tip of the handle. Such an instrument is proposed in the above-referenced Weber, Jr. 720 patent. While this arrangement may have some benefit, it still may be necessary to wipe off the tip of the handle once the blade is retracted. It is believed that a more direct and effective approach to the problem would be to reduce the amount of debris created during the electrocautery process, thereby eliminating or at least reducing the need to clean the electrode/blade.

US 5,195,959 describes an electrosurgical device with suction and irrigation, corresponding to the preamble of claim 1.

In view of the foregoing considerations, the present invention is directed to an improved electrocautery instrument.

The invention provides a fluid-assisted electrocautery instrument as defined in claim 1.

In one embodiment of the invention, an electrocautery instrument is configured with an electrode/blade disposed within a retractable suction tube, such that with the suction tube advanced, the electrode/blade is concealed within the tube, and with the suction tube retracted, the distal end of the electrode/blade is exposed for performing electrocautery.

In accordance with one embodiment of the invention, the electrocautery electrode/blade is implemented with a hollow, conductive tube, flattened at it distal end into a blade-like configuration. Conductive fluid is applied to the proximal end of the hollow electrode/blade, and expelled from the distal (blade) end thereof during electrocautery. In accordance with another embodiment of the invention, the conductive fluid emanating from the electrode/blade conducts the RF electrocautery energy away from the blade, so that it is primarily the fluid, rather than the metal blade, which actually accomplishes the cutting of tissue. That is, the fluid serves as a "virtual" electrocautery electrode. Since it is the fluid, rather than the blade, which incises and cauterizes, no burns or perforations are made to the tissue, reducing the amount of debris in the incision. Also, the flow of fluid through the electrode/blade tends to keep the blade clean and cool.

The foregoing and other aspects of the present invention may perhaps be best appreciated with reference to a detailed description of a specific embodiment of the invention, given by way of example only, when read in conjunction with the accompanying drawings, wherein:
Figure 1 is a perspective view of an electrocautery instrument in accordance with one embodiment of the invention; and
Figure 2 is a enlarged perspective view of the distal end of the electrode/blade of the electrocautery instrument of Figure 1.

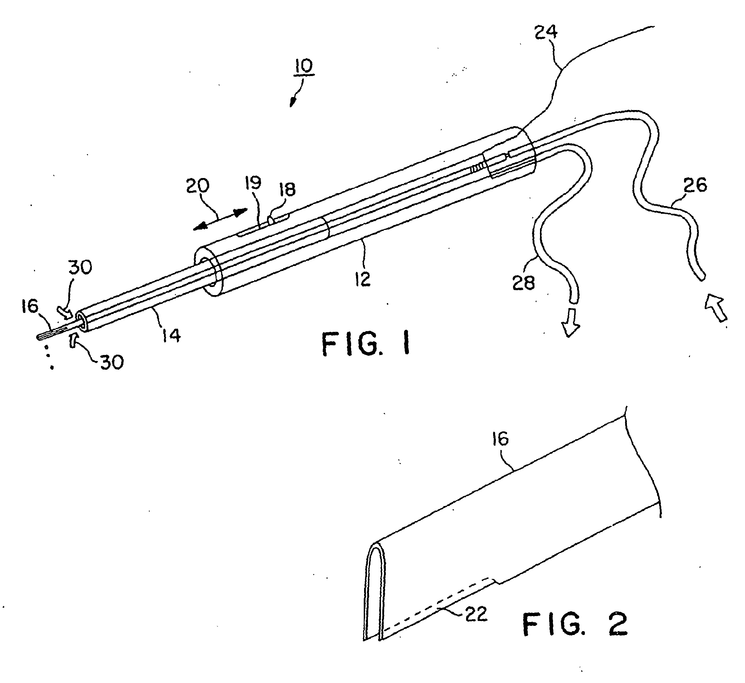

Referring to Figure 1, there is shown a perspective view of a fluid-assisted electrocautery instrument 10 in accordance with one embodiment of the invention. Electrocautery instrument 10 comprises a handle 12, a suction tube 14, and an electrocautery electrode/blade 16. Handle 12 is preferably made of a sterilizable, rigid, and non-conductive material, such as nylon or the like. Suction tube 14, which is also preferably made of a sterilizable and non-conductive material, is slidably disposed partially within an internal lumen of handle 12, and projects distally out of the end thereof. Electrode/blade 16 is disposed within suction tube 14 and handle 12. Suction tube 14 is adapted to slide proximally and distally with respect to handle 12 and electrode 16 (i.e., in the directions of arrow 20 in Figure 1) by means of a sliding lever 18 extending out of a slot 19 in handle 12. With suction tube 14 in a retracted position, as shown in Figure 1, a distal portion of electrode/blade 16 projects beyond the distal end of tube 14, such that electrocautery can be performed. With suction tube in an advanced position, suction tube 14 completely conceals the tip of electrode/blade 16.

In accordance with one embodiment of the invention, electrode/blade 16 is preferably implemented using a hollow cylindrical tube which has been flatted at its distal end, as shown in the greatly enlarged perspective view of Figure 2. In addition to being flattened, a portion of the distal end of electrode/blade 16 is removed to form a longitudinal slit 22 therein.

Three connections are made to electrocautery instrument 10: One terminal (e.g., positive) of a radio-frequency (RF) generator (not shown in Figure 1) is electrically coupled to electrode/blade 16 via a wire 24; a source of fluid to be expelled from slit 22 in electrode/blade 16 is coupled to the proximal end of electrode/blade 16 via a flexible tube or hose 26; and a suction hose 28 is coupled to handle 12 so as to be in communication with the internal lumen of handle 12 and with suction tube 14. When suction is applied via hose 28, air and fluid are drawn into the distal end of suction tube 14, as indicated by arrows 30. The ability to advance or retract suction tube 14 with respect to electrode/blade 16 enables the operator of the instrument to perform electrocautery while simultaneously aspirating smoke and fluid from the incision site, or to use suction tube 14 alone, without performing electrocautery.

As noted above, conductive fluid is communicated from inflow tube 26 and communicated along the length of electrode/blade 16 to be expelled from the distal end thereof. This is done in order to establish a so-called virtual electrode for performing electrocautery. The infusion of conductive fluid simultaneously with the application of RF energy is discussed in further detail in: US patent number 5,431,649, in US patent number 5,609,151; and in US patent number 5,876,398. The foregoing patents are hereinafter collectively referred to as "the RF ablation applications".

As described in the RF ablation patents, the infusion of conducting fluid into the area of application of RF energy creates a "virtual electrode," the size and shape of which can be controllably modified, and which can be rendered more or less conductive, thereby modifying the spread of RF energy. By varying such factors as the RF energy and duration, the rate of infusion of conductive liquid, and the conductivity of the infused solution, the size, shape, and intensity of the "virtual electrode" -- i.e., the intensity of thermal production in the area, can be controlled. In the case of the electrocautery device in accordance with the present invention, application of the conductive solution during the application of RF energy further assists by preventing overheating of the electrode/blade, extending the point at which burning or charring of tissue would otherwise normally occur. To enhance this effect, it is contemplated that the solution being infused may first be cooled.

Conductive solutions believed to be suitable for establishing the virtual electrode include saline, saturated saline, and Ringer's solution, among others. Regarding the source of conductive fluid, it is contemplated that a conventional pump may be coupled to input line 26. Alternatively, it is contemplated that a small, pre-pressurized canister of conductive solution may be used, such that no pump is required. In one embodiment, handle 12 may be configured to receive such a pressurized canister therein, eliminating the need for input line 26.

Although in the embodiment of Figure 1, input line 26, suction line 28, and electrical connection 24 are depicted separately, it is contemplated that these connections to instrument 10 may be consolidated into a single line having two separate fluid-conducting lumens therein (one for input of conductive solution, one for suction), alongside an insulated electrical conductor.

Various alternative configurations of electrode/blade 16 are also contemplated. In one embodiment, a porous metal element is substituted for the flattened tube configuration of Figures 1 and 2.

From the foregoing detailed description of a specific embodiment of the invention, it should be apparent that a method and apparatus for performing fluid-assisted electrocautery of body tissue has been disclosed, wherein fluid delivered out of a hollow electrocautery electrode/blade creates a virtual electrode which incises and cauterizes the tissue.

## Claims

1. A fluid assisted electrocautery device (10) comprising:
a non-conductive handle (12) defining a proximal end and a distal end and having a longitudinal lumen extending between said proximal and distal ends; and
a suction tube (14), disposed partially within said lumen of said handle and having a distal end extending out of said distal end of said handle;
a rigid, elongated electrode (16) adapted to be coupled to a source of radiofrequency energy extending distal the distal end of the handle, said electrode comprising an elongate tube defining an internal lumen extending between proximal and distal ends of said electrode,
a fluid input tube (26) coupled to said proximal end of said electrode and in fluid communication with the internal lumen of the electrode (16), such that conductive fluid supplied from said input tube is communicated along said electrode and expelled from said distal end of said electrode; **characterised by** said electrode being disposed within said suction tube such that a distal end of said electrode extends distally beyond said distal end of said suction tube and by said electrode having a porous metal element defining a distal end of the electrode, the electrode defining a lumen in fluid communication with the porous metal element, wherein the porous metal element is adapted to expel fluid from the distal end of the electrode.

2. The device (10) of claim 1, wherein the electrode (16) is rigidly connected to the handle (12).

3. The device (10) of claim 1, wherein at least a portion of the electrode (16) is co-axially disposed within the handle (12).

4. The device (10) of claim 1, further comprising:
a non-conductive suction tube (14) co-axially disposed over at least a portion of the electrode (16) distal the distal end of the handle (12).

5. The device (10) of claim 4, wherein the non-conductive suction tube (14) defines an outermost surface of the medical device immediately distal the handle (12).

6. The device (10) of claim 1, wherein at least one passage is formed in the porous metal element.

7. The device of claim 1, wherein the fluid input tube (26) extends within the handle (12).

8. A system comprising:
the device (10) of claim 1;
a conductive fluid source fluidly connected to the electrode lumen; and
a radiofrequency energy source electrically connected to the electrode;
wherein the porous metal element is adapted to expel conductive fluid from the distal end of the electrode and to apply radiofrequency energy to the distributed fluid.

9. The medical system of claim 8, wherein the electrode (16) is rigidly connected to the handle (12).

10. The medical system of claim 8, wherein at least a portion of the electrode (16) is co-axially disposed within the handle (12).

11. The medical system of claim 8, wherein at least one passage is formed in the porous metal element.

12. The medical system of claim 8, wherein:
said fluid input tube (26) fluidly connects the conductive fluid source and the electrode (16) lumen.

13. The medical system of claim 12, wherein the fluid input tube (26) extends within the handle (12).

## Patentansprüche

1. Vorrichtung (10) zur fluidunterstützten Elektrokauterisation mit:
einem nichtleitenden Griff (12), der ein Proximalende und ein Distalende definiert und ein Längslumen aufweist, das sich zwischen dem Proximalende und dem Distalende erstreckt; und
einem Saugrohr (14), das teilweise in dem Lumen des Griffs angeordnet ist und ein Distalende aufweist, das sich aus dem Distalende des Griffs heraus erstreckt;
eine starre, längliche Elektrode (16), die dafür eingerichtet ist, mit einer Hochfrequenzenergiequelle verbunden zu werden, und die sich distal des Distalendes des Griffs erstreckt, wobei die Elektrode ein Längsrohr aufweist, das ein Innenlumen definiert, das sich zwischen einem Proximalende und einem Distalende der Elektrode erstreckt,
ein Fluideingaberohr (26), das mit dem Proximalende der Elektrode verbunden ist und in fluidischer Verbindung mit dem Innenlumen der Elektrode (16) steht, so dass leitendes Fluid, das von dem Eingaberohr bereitgestellt wird, entlang der Elektrode weitergegeben und aus dem Distalende der Elektrode ausgestoßen wird; **dadurch gekennzeichnet, dass** die Elektrode in dem Saugrohr so angeordnet ist, dass ein Distalende der Elektrode sich distal über das Distalende des Saugrohrs hinaus erstreckt, und dass die Elektrode ein poröses Metallelement aufweist, das ein Distalende der Elektrode definiert, wobei die Elektrode ein Lumen in fluidischer Verbindung mit dem porösen Metallelement definiert, wobei das poröse Metallelement dafür eingerichtet ist, Fluid aus dem Distalende der Elektrode auszustoßen.

2. Vorrichtung (10) nach Anspruch 1, bei der die Elektrode (16) starr mit dem Griff (12) verbunden ist.

3. Vorrichtung (10) nach Anspruch 1, bei der wenigstens ein Teil der Elektrode (16) koaxial in dem Griff (12) angeordnet ist.

4. Vorrichtung (10) nach Anspruch 1, ferner mit:
einem nichtleitenden Saugrohr (14), das koaxial über wenigstens einem Abschnitt der Elektrode (16) distal zu dem Distalende des Griffs (12) angeordnet ist.

5. Vorrichtung (10) nach Anspruch 4, bei der das nichtleitende Saugrohr (14) eine äußerste Oberfläche der medizinischen Vorrichtung unmittelbar distal des Griffs (12) definiert.

6. Vorrichtung (10) nach Anspruch 1, bei der wenigstens ein Durchgang in dem porösen Metallelement ausgebildet ist.

7. Vorrichtung (10) nach Anspruch 1, bei der das Fluideingaberohr (26) sich in dem Griff (12) erstreckt.

8. System mit:
der Vorrichtung (10) nach Anspruch 1;
einer Quelle für leitendes Fluid, die in fluidischer Verbindung mit dem Elektrodenlumen steht; und
einer Hochfrequenzenergiequelle, die elektrisch mit der Elektrode verbunden ist;
wobei das poröse Metallelement dafür eingerichtet ist, leitendes Fluid aus dem Distalende der Elektrode auszustoßen um Hochfrequenzenergie auf das verteilte Fluid aufzubringen.

9. Medizinisches System nach Anspruch 8, bei dem die Elektrode (16) starr mit dem Griff (12) verbunden ist.

10. Medizinisches System nach Anspruch 8, bei dem wenigstens ein Teil der Elektrode (16) koaxial in dem Griff (12) angeordnet ist.

11. Medizinisches System nach Anspruch 8, bei dem wenigstens ein Durchgang in dem porösen Metallelement ausgebildet ist.

12. Medizinisches System nach Anspruch 8, bei dem:
das Fluideingaberohr (26) die Quelle für leitendes Fluid und das Lumen der Elektrode (16) fluidisch verbindet.

13. Medizinisches System nach Anspruch 12, bei dem das Fluideingaberohr (26) sich in den Griff (12) erstreckt.

## Revendications

1. Dispositif d'électrocautérisation assisté par fluide (10) comportant :
une poignée non conductrice (12) définissant une extrémité proximale et une extrémité distale et ayant une lumière longitudinale s'étendant entre lesdites extrémités proximale et distale ; et
un tube d'aspiration (14), disposé partiellement dans ladite lumière de ladite poignée et ayant une extrémité distale s'étendant hors de ladite extrémité distale de ladite poignée ;
une électrode rigide, allongée (16) adaptée pour être couplée à une source d'énergie radiofréquence s'étendant de manière distale par rapport à l'extrémité distale de la poignée, ladite électrode comportant un tube allongé définissant une lumière interne s'étendant entre les extrémités proximale et distale de ladite électrode,
un tube d'entrée de fluide (26) couplé à ladite extrémité proximale de ladite électrode et en communication fluide avec la lumière interne de l'électrode (16), de sorte que le fluide conducteur délivré depuis ledit tube d'entrée est communiqué le long de ladite électrode et expulsé par ladite extrémité distale de ladite électrode ; **caractérisé en ce que** ladite électrode est disposée dans ledit tube d'aspiration de sorte qu'une extrémité distale de ladite électrode s'étend de manière distale au-delà de ladite extrémité distale dudit tube d'aspiration ; et **en ce que** ladite électrode comporte un élément métallique poreux définissant une extrémité distale de l'électrode, l'électrode définissant une lumière en communication fluide avec l'élément métallique poreux, dans lequel l'élément métallique poreux est adapté pour expulser le fluide de l'extrémité distale de l'électrode.

2. Dispositif (10) selon la revendication 1, dans lequel l'électrode (16) est connectée de manière rigide à la poignée (12).

3. Dispositif (10) selon la revendication 1, dans lequel au moins une partie de l'électrode (16) est disposée de manière coaxiale dans la poignée (12).

4. Dispositif (10) selon la revendication 1, comportant en outre :
un tube d'aspiration non conducteur (14) disposé de manière coaxiale sur au moins une partie de l'électrode (16) distale de l'extrémité distale de la poignée (12).

5. Dispositif (10) selon la revendication 4, dans lequel le tube d'aspiration non conducteur (14) définit une surface située le plus à l'extérieur du dispositif médical immédiatement distale par rapport à la poignée (12).

6. Dispositif (10) selon la revendication 1, dans lequel au moins un passage est formé dans l'élément métallique poreux.

7. Dispositif selon la revendication 1, dans lequel le tube d'entrée de fluide (26) s'étend dans la poignée (12).

8. Système comportant :
le dispositif (10) de la revendication 1 ;
une source de fluide conductrice reliée de manière fluide à la lumière de l'électrode ; et
une source d'énergie radiofréquence reliée électriquement à l'électrode ;
dans lequel l'élément métallique poreux est adapté pour expulser le fluide conducteur de l'extrémité distale de l'électrode et pour appliquer une énergie radiofréquence au fluide distribué.

9. Système médical selon la revendication 8, dans lequel l'électrode (16) est reliée de manière rigide à la poignée (12).

10. Système médical selon la revendication 8, dans lequel au moins une partie de l'électrode (16) est disposée de manière coaxiale dans la poignée (12).

11. Système médical selon la revendication 8, dans lequel au moins un passage est formé dans l'élément métallique poreux.

12. Système médical selon la revendication 8, dans lequel :
ledit tube d'entrée de fluide (26) relie de manière fluide la source de fluide conductrice et la lumière d'électrode (16).

13. Système médical selon la revendication 12, dans lequel le tube d'entrée de fluide (26) s'étend dans la poignée (12).
